# NEW EUROPEAN PATENT SPECIFICATION

(11) **EP 0 527 917 B2**
(45) Date of publication and mention of the opposition decision: **16.08.2006**
(45) Mention of the grant of the patent: 21.01.1998
(21) Application number: 91909913.5
(22) Date of filing: 07.05.1991
(51) Int. Cl.: A61K 31/70

(54) **USE OF ADENOSINE AND ADENOSINE DERIVATIVES FOR ANESTHESIA**
VERWENDUNG VON ADENOSINE UND ADENOSINEDERIVATE ZUR ANÄSTHESIE
ADENOSINE ET DERIVES DE ADENOSINE UTILISES DANS DES PROCESSUS D'ANESTHESIE

(30) Priority: 10.05.1990 US 521529
(43) Date of publication of application: 24.02.1993
(60) Divisional application: 97107216.0
(73) Proprietor: FUKUNAGA, Atsuo F., Rancho Palos Verdes, CA 90274 (US)
(72) Inventor: FUKUNAGA, Atsuo F., Rancho Palos Verdes, CA 90274 (US)
(74) Representative: Vossius & Partner
(86) International application number: PCT/US1991/002951
(87) International publication number: WO 1991/016903

(56) References cited:
- GB-A- 2 171 305
- US-A- 5 677 290
- Revista Espanola de Anestesiologia y Reanimacion, vol. 33, no. 1, February 1986, ES; F. GONZALES MIRANDA et al.: "Implicaciones anestesicas del sistema purinergico: revision", pages 25-28, see abstract
- Pain, vol. 25, no. 2, May 1986, Amsterdam, NL; J.D. LOESER: "Herpes zoster postherpetic neuralgia", pp. 149-164
- Anesthesiology, vol. 70, no. 2, February 1989, Am. Soc. of Anesthesiologists, Inc., US; M. DOI et al.: "Sevoflurane anesthesia with adenosine triphosphate for resection of pheochromocytoma", pp. 360-363
- Biology of the Neonate, vol. 47, no. 6, 1985, Basel, CH; T. HEDNER et al.: "Characterization of adenosine-induced respiratory depression in the preterm rabbit", pages 323-332, see abstract
- Journal of Pediatric Surgery, vol. 23, no. 12, December 1988, Grune & Stratton, US; C.N. PAIDAS et al.: "Adenosine triphosphate: a potential therapy for hypoxic pulmonary hypertension", pages 1154-1160, see abstract
- Biochem. Med. Metab. Biol., vol. 38, no. 3, December 1987, US; J. NORDENBERG et al.: "Exogenous ATP antagonizes the actions of phospholipase A2, local anesthetics, Ca2+ ionophore A23187, and lithium on glucose-1,6-bisphosphate levels and the activities of phosphofructokinase and phosphoglucomutase in rat muscle", pages 278-291, see page 284
- ANAESTESIOLOGY, vol. 71, no. 3A, Sep 1989, abstract A260
- J Am Coll Cardiol, vol 23, no 2, Feb 1994, pp. 384-389
- Am J Cariology, vol 72 no 14,1993, pp. 983-989
- J Am Coll Cardiol, vol 16, no. 6, 1990, pp. 1375-1383
- Circulation, vol 87, no. 2, 1993, pp. 345-354
- S. Gröndal et al., World J. Surg., 12 (1988) pp. 131-138

## Description

### BACKGROUND OF THE INVENTION

### Field of the Invention

This invention relates to uses of adenosine and adenine nucleotides as anesthetic agents.

### Relevant Art

A patient is protected from the pain and stress of surgery and similar procedures, by anesthesia to maintain physiological homeostasis.

Adenosine has a variety of extracellular effects. It is known to have potent vasodilating and hypotensive effects, but it has never been demonstrated that it has anesthetic activity with dinical use. Furthermore, the conventional wisdom is that neither adenosine nor adenosine triphosphate (hereinafter ATP), an adenine nucleotide, circulating in the blood will cross the blood brain barrier. Therefore, despite known sedative activity of some synthetic adenosine analogues, neither adenosine nor ATP had been ever thought to be suitable as anesthetics.

A variety of drugs are used to provide anesthesia. Goodman and Gilman's The Pharmacological Basis of Therapeutics, 7th Ed., 1985, MacMillan, New York, Chapters 13 and 14 provide an overview of the field of anesthesiology as currently understood by those skilled in the art.

It has been reported that adenosine can be used in conjunction with a standard inhalational anesthetic to reduce the amount of anesthetic required, when adenosine is administered to produce hypotension. But others have never reported that adenosine or ATP could produce substantially acceptable anesthesia under normotensive conditions or could totally replace an inhalational anesthetic and still achieve substantially acceptable anesthesia.

Total replacement or reduction of inhalational agents or other agents is advantageous for several reasons. First, inhalational or other synthetic chemical anesthetics are toxic in large doses and produce severe cardio-respiratory and metabolic side effects. Secondly, the amount of anesthetic actually being used by the patient is subject to guesswork in the operating room.

Adenosine and ATP are endogenous compounds and therefore unlikely to produce toxic effects. Both Adenosine and ATP are known to be rapidly metabolized; therefore when the infusion is stopped, recovery starts immediately and proceeds rapidly. Therefore, either adenosine or ATP would be ideal replacements for inhalational anesthetics.

Applicant has found that under normotension, intravenously administered adenosine and ATP have potent analgesic effects, which together with the sedative effects and the inhibition of stress response effects, such as antihypertension and blood pressure control effects, make it a superior anesthetic agent.

Furthermore, when a subject anesthetized with adenosine or ATP and is then subjected to body temperature decrease, the decrease is not accompanied by shivering, cardiovascular distress, or pulmonary distress. This effect holds for drops in body temperature at least as large as 10°C to 20°C.

### SUMMARY OF THE INVENTION

This invention provides the use of adenosine or an adenine nucleotide for the preparation of an anesthetic composition.

The anestetic composition of the invention may be used for anesthetizing an individual of a mammalian species by administering it as a continuous dependent infusion of between 1 µg/kg/min and 5000 µg/kg/min of adenosine or an adenine nucleotide for the period that anesthesia is desired.

### BRIEF DESCRIPTION OF THE DRAWINGS

FIGURE 1 shows a chart comparing the activities of several different classes of anesthetics.
FIGURE 2 shows a graphical representation of the amount of anesthetic effect, measured as halothane MAC, versus the dosage of adenosine or ATP administered.
FIGURE 3 shows a graphical representation of the dose response curve of the anesthetic effect of ATP versus the nociceptive electrical stimulation threshold (V), and tail clamp (ED50)
FIGURE 4 shows a graphical representation of the amount of anesthetic effect, measured as enflurane MAC, versus the dosage of ATP administered.

### DETAILED DESCRIPTION OF THE PREFERRED EMBODIMENT

### Definitions

As used herein "anesthesia" is defined as the final result of several interacting but independent effects. The first effect is sedation and/or sleep induction; the second is analgesia or pain relief; the third is stress reduction to preserve physiological homeostasis, most frequently seen as blood pressure modification during surgery; and finally, the fourth is usually considered to be muscle relaxation, particularly relaxation of skeletal muscle. At the present time, no single agent provides adequate levels of each and all of these four effects, so combinations of drugs must be used in cases like surgery. Anesthetic as used herein will refer to any single drug that gives rise to at least two of the four effects.

As used herein, the "adenine nucleotides" are only adenosine monophosphate, adenosine diphosphate, and adenosine triphosphate. In general, the preferred adenine nucleotide is adenosine triphosphate.

As used herein "surgery" and "surgical procedures" refer broadly to invasive discomfort-producing medical procedures. Included are such procedures as endoscopy, angiography, dental work, such as tooth extractions, as well as what is traditionally thought of a surgery, for example appendectomies and the like. As used herein the terms encompass the presurgical phase and the post surgical phase as well, for example the emergency room, post-anesthesia care room, the intensive care unit, and the like, until the patient can rest comfortably without the supplemental administrations of analgesics being indicated.

As used herein "continuous infusion" refers to intravascular infusions, intrathecal infusions, and like methods of providing a continuous drug dosage over a period of time.

As used herein "stress" refers to the physiological changes that accompany trauma such as surgery. It includes release of catecholamines, induction of hypertension, and vasoconstriction.

Adenosine is known to have a short plasma half life. In order to achieve the process of this invention safely, a continuous infusion of adenosine or an adenine nudeotide must be used. An infusion of approximately 1 to 5500 µg of adenosine/kg bodyweight/min, hereinafter µg/kg/min, more preferably, 5 to 1000 µg/kg/min, even more preferably between 50 to 500 µg/kg/min, provides the effect of the invention.

The anesthetic effects of this invention are compared to standard anesthetic effects by comparing minimum alveolar concentration (hereinafter MAC). A measure of potency of anesthetics has proven to be elusive. A first problem is only a relatively small amount of inhalational anesthetic gases is absorbed before the gas is exhaled. A second problem is that the point of action of the anesthetic is the brain, not the lungs, and the amount of anesthetic transported by blood to the brain, and the amount that actually enters brain tissue, is unknown resulting in an ill defined concentration of gas in brain tissue. Therefore, those skilled in the art of anesthesiology define one MAC of a gaseous anesthetic at 1 atmosphere of pressure (760 torrs) as that amount which produces immobility in 50 percent of patients or animals exposed to painful stimulus. The use of MAC provides a convenient means of comparing the effect of the standard gaseous anesthetic agents, halothane, enflurane, methoxyflurane, isoflurane, and nitrous oxide to the anesthetic effect of adenosine and adenine nucleotides.

The anesthetic and analgesic properties of adenosine and adenine nucleotides are more easily compared to the effective dose of opioid analgesics. There, a dosage of opioid sufficient to produce immobility in 50 percent of patients or animals exposed to painful stimuli is defined as the effective dose. (hereinafter ED 50)

Blood pressure can be lowered by administration of adenosine or adenine nucleotides, and other means, for example hemorrhage. The overall response to blood pressure lowering to a predetermined level by administration of purines and hemorrhage is quite different. In the case of hemorrhage, oxygen in all tissues and organs plunges, but in the case of purine infusion the levels actually increase. As a result, administration of adenosine and adenine nudeotides can increase the oxygen content of tissues.

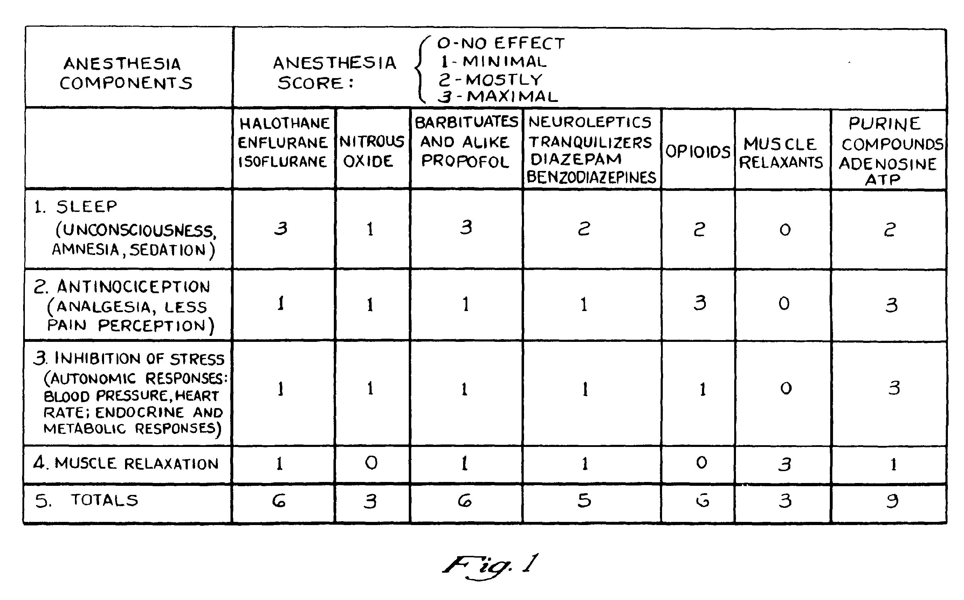

Referring to Figure 1 various anesthetics and anesthetic agents are compared for efficacy in producing sleep, inducing analgesia, reducing surgical stress and relaxing muscles. These are the four effects that anesthesiologists try to achieve during a surgical procedure. The chart confirms that no presently used agent is effective for all four effects. All known agents for stress relief are not included in the chart because most of these agents are normally considered to be cardiovascular medications, given primarily to treat cardiovascular disease, rather than anesthetic agents.

On the chart, a class of agents is rated for each of the four effects and graded on a scale of 0, indicating no effect, to 3, indicating a large effect.

It can be seen that sleep is best sustained by the inhalation agents: halothane, and the like; and induced by barbiturates: sodium pentothal, and the like, and propofol. Pain is most effectively relieved by the opioid analgesics: morphine, fentanyl, and the like. Muscle relaxation is induced by a paralytic agent, pancuronium, succinylcholine chloride, and the like. But each agent has two or more points of poor effectiveness, as shown by a 0 or 1 on the chart. As a result, in a surgical procedure an anesthesiologist must use several different agents to achieve successful anesthesia. In particular, no currently used anesthetic agent has much effectiveness against the stress induced by surgery.

Adenosine or adenine nucleotides, on the other hand are seen to provide excellent analgesia, and stress relief, good sedation induction, and maintenance, but only fair muscle relaxation.

An ideal anesthetic for surgical use would combine several effects in one medication. It would provide pain relief, sedation and sleep, and induce muscle relaxation. Typically today an anesthesiologist must relieve pain by an opioid or other analgesic, produce sleep by sodium pentothal or similar hypnotic, sustain sleep by an inhalational anesthetic and relax muscles by the use of succinyl choline chloride or the like.

Adenosine and adenine nudeotides induce sedation, maintain sleep, reduce stress of surgery, and relieve pain. In addition, adenosine can induce hypothermia and maintain tissue perfusion and oxygenation. Therefore, adenosine and adenine nucleotides come closer to being ideal anesthetics for surgical use.

Adenosine or adenine nucleotides may be the only agent required for certain uses, such as relief of chronic pain, or minor surgery where deep sleep is not necessary, but pain relief is. Major surgery may require the additional use of some inhalational or intravenous anesthetics and some muscle relaxant.

### Dosage

In contrast to other conventional anesthetics, adenosine and adenine nucleotides are useful over a wide dosage range. Adenosine is active over a range of between 1 to 5000 µg/kg/min. In contrast, conventional anesthetic agents, for example, isoflurane have narrow effective ranges. The fatal to anesthetic ratio of isoflurane is 3.02 ± 0.13. Other reports indicate that isoflurane has about 1.88 times the safety range of halothane. When these ranges are stated simplistically, they suggest that if a patient is administered between two and five times the dose required for the onset of sleep, the patient will die. Of course, the anesthesiologist will err on the side of too little, running the risk of under dosing the patient, which results in a subanesthetized patient during the surgical procedure.

One great advantage observed with adenosine is the wide margin for error. The lethal dose for adenosine is about 100 times the dose required for analgesia, and in the case of ATP is greater than 150 times the amount required for analgesia. In fact, in the case of ATP the ratio remains unknown since no experimental animals died even given enormous overdoses.

### Anesthetic Effects

The anesthetic effect of adenosine and adenine nucleotides is measured using New Zealand white rabbits having shaved tails. Intravenous drugs are introduced in the rabbits ears. The rabbits breathed halothane in O₂ through a face mask. The rabbits can be intubated, but are usually observed to breathe spontaneously. MAC is determined by clamping the proximal one-third of the tail with a rubber shod hemostat. The anesthetic effect of adenosine is determined by reducing the amount of halothane from 1.00 MAC to 0.75 MAC to 0.50 MAC to 0.25 MAC to 0 MAC while increasing the infusion rate of adenosine at a level to achieve an observed 1.00 MAC of anesthesia. Temperature of the rabbits is maintained at between 38.5°C and 39.5°C. By use of similar techniques the analgesic effects of adenosine can be compared to those of morphine. The dose response curve of the analgesic effects of adenosine and ATP can also be obtained by the method described above.

### Formulations

Adenosine or adenine nucleotides can be prepared for intravenous solution by conventional methods known in the art. Pharmaceutically acceptable excipient and adjuvants, for example, salts, sugars, pH modifiers, thickeners and the like as well as other pharmaceuticals with similar or different activities can be added to achieve a clinically acceptable dosage form. All these modifications are well within the scope of knowledge of one reasonably skilled in the art.

### EXAMPLES

Adenosine triphosphate - Na₂ was obtained from Kyowa Hakko, Japan. Enough ATP was dissolved in physiologic saline to make a 200 mg/ml solution. Adenosine solution was made dissolving adenosine (Sigma Co.) in physiological saline to make a 10 mg/ml solution, and adenosine solution (5.3 mg/ml in isotonic mannitol) was obtained from Astra Co.

### EXAMPLE 1

This example shows the effects of intravenously administering adenosine and ATP on Halothane MAC and its reversal by aminophylline in rabbits.

Adenosine is known to produce a number of effects in the CNS including sedation, anticonvulsant and antinociceptive (analgesic) effects. We studied the effect of an administration of an intravenous infusion of adenosine and ATP on MAC on halothane in rabbits.

Anesthesia was induced in 23 unmedicated New Zealand white rabbits (4.5-5.5 Kg) with 3 to 4 percent halothane in O₂ using face mask; the trachea was intubated with a pediatric endotracheal tube. Each animal breathed spontaneously throughout the study. Esophageal temperature was kept at 38°5-39.5°C. Endo-tidal halothane and CO₂ were monitored continuously. Two ear veins and an ear central artery were cannulated with 22 gauge plastic catheters for drug infusion, and direct blood pressure and gas monitoring. There were 4 groups: A (n=7) treated with phenylephrine to support blood pressure during ATP, B (n=7) ATP alone, C (n=5) adenosine alone, D (n=4) ATP combined with dipyridamole. MAC was determined using the standard procedure of clamping the proximal one-third of the shaved tail with a rubber shod hemostat closed to first ratchet.

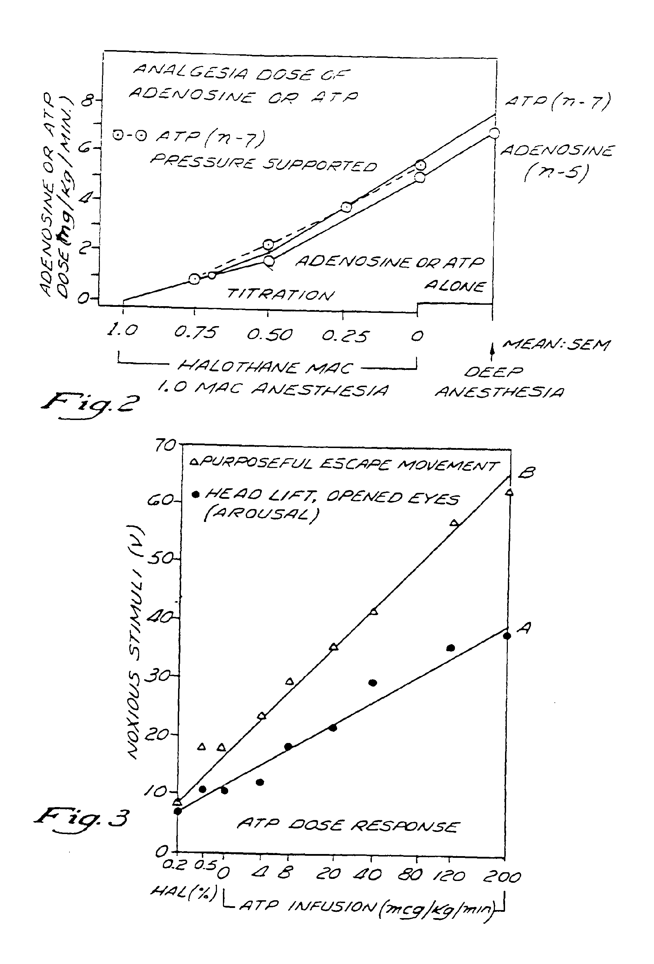

Referring to Figure 2, the analgesic level of adenosine or ATP was estimated by, titrating the drug infusion rate step wise while decreasing the amount of halothane MAC from 1.0 - 0.75 - 0.25 - 0. Halothane MAC values in all 4 groups averaged 1.62±0.56%, with no significant difference between them. Both drugs produced a potent analgesic action which was dose-related; this was potentiated by dipyridamole in both pressure supported normotensive and hypotensive groups. At 5.52±0.79 mg/kg/min for ATP or 5.32±0.82 mg/kg/min for adenosine, halothane anesthesia could be entirely replaced. When maintained from 30 minutes to 1 hour, the animals were deeply sedated and become completely insensitive to any painful stimulus. The anesthetic response was for greater than 1 MAC. As the dose was increased the anesthetic effect became deeper and deeper.

Administration of aminophylline (50-100 mg IV) and naloxone (0.01-0.05 mg IV) rapidly reversed the anesthetic effect, and the animals resume normal activity within 5 minutes without any abnormal neuro-behavioral sign.

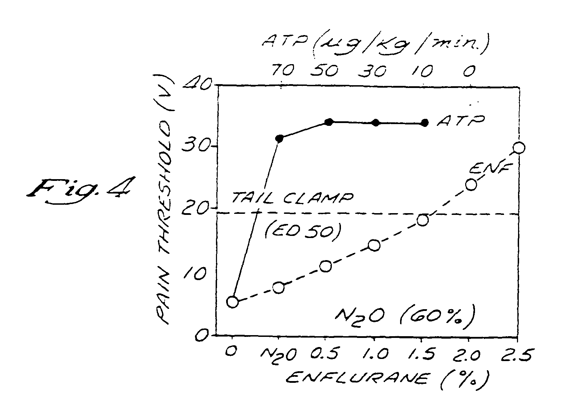

Referring to Figure 3, an ATP infusion is slowly increased. The amount of electrical stimulation required to elicit a response is noted. ATP shows selective responses. Two easily differentiated threshold responses are seen: one (A) is a response to arousal, and the second (B) is a purposeful escape movement in response to perceived pain.

Both ATP and adenosine alone had a potent anesthetic action which could totally replace halothane MAC. It is known that ATP rapidly degrades to adenosine; therefore, we infer that profound analgesia produced by ATP or adenosine appear to mediate central adenosine receptors since both drugs equally attained comparable effects of sedation, analgesia and anesthesia which were potentiated by dipyridamole, an adenosine uptake inhibitor, and reversed by aminophylline, an adenosine antagonist.

### EXAMPLE 2

Adenosine reduces MAC requirements for enflurane. High doses of enflurane (ENF) with N₂O attenuates responses to noxious stimuli, but at the cost of cardio-respiratory depression. In this example ATP is substituted for ENF. It provides sufficient anesthesia while avoiding the cost of cardio-respiratory depression. 6 intubated rabbits (4-5Kg) spontaneously breathing 60% N₂O in O₂ were studied. Electrical stimulation in increasing voltage, as tolerated, up to 50v was applied. This allowed quantifiable, reproducible stimulation. Measurements were taken every 15 min. Purposeful escape movements were used as end-points. ENF concentrations of 0.5, 1.0, 1.5, 2.0, 2.5% were added to N₂O stepwise; responses and blood gases were recorded; in addition to electrical stimulation, the tail, ear and leg were clamped with a hemostat, and pinprick was applied to reconfirm responses to nodception. When negative response to stimuli was achieved, the dose of ENF was decreased stepwise by 0.5% till positive response was shown; then, ATP infusion was titrated to replace the decreased ENF anesthesia till ATP could totally and effectively replace ENF (ATP initial dose: 5 µg/kg/min).

Referring to Figure 4 under 60% N₂O, increasing doses of ENF up to 1.5% showed an elevated pain-threshold, but still responded positively to tail clamp. ENF, more than 2%, could completely inhibit such responses. At this dose, however, significant cardio-respiratory depression was shown (see Table 1). Addition of increasing doses of ATP (5-70 µg/kg/min) allowed ENF to be replaced without diminishing the pain tolerance. Moreover, systemic blood pressure (SBP) and heart rate (HR) returned to control levels but analgesia persisted. Although Adenosine and ATP have extremely short plasma half lives, IV ATP may have intrinsic analgesic activity in the CNS since the selective analgesic effect persisted after the infusion had stopped; this effect may have been due to activation of central purinergic receptor mechanism, which once activated, has a long duration. This was seen by the observation of sustained analgesia after discontinuation of ATP which was partially reversed by IV aminophylline. This sustained analgesic property without cardio-respiratory depression may have marked clinical significance.

**TABLE 1**

| Cardio-Respiratory Data | | | | |
|---|---|---|---|---|
| | O₂ ONLY | CONTROL (N₂O) | ENF (N₂O) | ATP (N₂O) |
| SBP(mmHg) | 109±11 | 126±15 | 69±22* | 112±20 |
| DBP(mmHg) | 83±5 | 85±4 | 49±18* | 83±16 |
| HR(bpm) | 270±13 | 267±15 | 255±33* | 278±26 |
| RR(bpm) | 76±5 | 85±5 | 72±19* | 81±14 |
| PaCO₂(mmHg) | 25±4 | 22±2 | 25±1* | 22±3 |
| PaO₂(mmHg) | 443±68 | 147±11 | 148±16 | 145±24 |
| RR: Respiratory Rate, O₂:100%, N₂O:60%, | | | | |
| ENF: 2.0-2.5%, ATP: 70±32(ug/kg/min), Mean±SD | | | | |

| | | | | |
|---|---|---|---|---|
| p<0.05 vs CONTROL (n=6) | | | | |

### EXAMPLE 3

In this example, intrathecal administration of adenosine is shown to be effective.

Anesthesia is induced in unmedicated New Zealand white rabbits (4-5 Kg) with 3-4% halothane in oxygen using a face mask. The trachea is intubated with a No. 3.5 pediatric endotracheal tube. All the animals breathe spontaneously throughout the study but they are mechanically ventilated when required. Rectal temperature are controlled at 38.5-39.5 °C. Expired halothane and carbon dioxide concentrations are monitored continuously. Two ear veins and an ear central artery are cannulated with 22 gauge plastic catheters for drug infusion and for blood gas monitoring. The groin is exposed and a cut-down performed on the femoral artery and vein. The femoral artery is cannulated and the catheter placed with its tip in the mid-thoracic aorta to measure central systemic blood pressures. Another central venous catheter is inserted through the femoral vein.

The animal is turned in prone position and the operative site prepared over the lower back. The skin is infiltrated with 2% lidocaine 3-4 cc. Following dissection through the paravertebral muscles, a laminectomy is performed, a touhy needle is placed under direct vision into sub-arachnoid space and a microcatheter (22-32 gauge) is threaded through the needle advancing it gently so that its tip lays at the base of the skull. Clear CSF is aspirated to confirm sub-arachnoid placement. The laminectomy site is disinfected, and sutured to close the site. The catheter is secured with tape.

Noxious stimulation is provided by an electrode placed at the base of the tail for low-voltage electrical current stimulation delivered through a nerve stimulator. Other kind of stimulations will be in the form of pin prick with a 20 gauge needle, pinching the paw and tail clamping with a rubber-shod hemostat.

Continuous recording of cardio-respiratory parameters including the heart rate, arterial blood pressure, respiratory rate, the animal's physical movement, and any other behavioral changes are recorded. Samples of arterial and mixed venous blood are analyzed for pulmonary gas exchange and acid base status.

Several intrathecal dosage forms are evaluated: Lidocaine is given in 0.5 mg/kg increments. Fentanyl is given in 1 µg/kg increments; Adenosine is given as a fractionated dose as well as via a continuous infusion (10 mg/hr) based on the body weight of the rabbit.

Comparison of the various drugs show that intrathecal administration of adenosine is as effective as intrathecal fentanyl in reducing the response to the noxious stimulation.

## Claims

1. Use of adenosine, adenosine monophosphate, adenosine diphosphate, or adenosine triphosphate for the preparation of a composition for anaesthetizing an individual of a mammalian species during noxious stimulation during surgey **characterized in that** said composition provides protection from pain or stress induced by the noxious stimulation, without causing cardio-respiratory depression.

2. Use according to claim 1 wherein said composition is administrable in a continuous infusion of between 1 µg/kg/min and 5000 µg/kg/min of said composition for the period that anaesthesia is desired.

3. Use according to claims 1 or 2, wherein the composition is administrable via continuous intrathecal infusion.

4. Use according to claims 1 or 2, wherein the composition is administrable via continuous fractionated doses.

5. Use according to claims 1 or 2, wherein said pharmaceutical composition is administrable in a continuous intravascular infusion.

## Patentansprüche

1. Verwendung von Adenosin, Adenosinmonophosphat, Adenosindiphosphat oder Adenosintriphosphat für die Herstellung eines Mittels zur Anästhesie eines Säugers während einer schädlichen Stimulierung während eines operativen Eingriffs, **dadurch gekennzeichnet, dass** das Mittel vor Schmerz und Stress schützt, die durch die schädliche Stimulierung hervorgerufen werden, ohne eine Verminderung der kardiorespiratorischen Funktion zu verursachen.

2. Verwendung nach Anspruch 1, wobei das Mittel über eine kontinuierliche Infusion in einer Menge von 1 µg/kg/min bis 5000 µg/kg/min des Mittels für den gewünschten Zeitraum der Anästhesie verabreicht werden kann.

3. Verwendung nach Anspruch 1 oder 2, wobei das Mittel über eine kontinuierliche intrathekale Infusion verabreicht werden kann.

4. Verwendung nach Anspruch 1 oder 2, wobei das Mittel kontinuierlich über fraktionierte Dosen verabreicht werden kann.

5. Verwendung nach Anspruch 1 oder 2, wobei das Arzneimittel über eine kontinuierliche intravaskuläre Infusion verabreicht werden kann.

## Revendications

1. Utilisation d'adénosine, d'adénosine-monophosphate, d'adénosine-diphosphate ou d'adénosine-triphosphate pour la préparation d'une composition destinée à l'anesthésie d'un individu d'une espèce mammalienne pendant une stimulation nocive au cours d'une chirurgie, **caractérisée en ce que** ladite composition assure une protection contre la douleur ou le stress induits par la stimulation nocive, sans provoquer de dépression cardio-respiratoire.

2. Utilisation selon la revendication 1, où ladite composition est administrable en perfusion continue à raison de 1 µg/kg/min à 5000 µg/kg/min de ladite composition pendant la durée où l'anesthésie est désirée.

3. Utilisation selon la revendication 1 ou 2, où la composition est administrablé par perfusion intrathécale continue.

4. Utilisation selon la revendication 1 ou 2, où la composition est administrable par doses fractionnées continues.

5. Utilisation selon la revendication 1 ou 2, où ladite composition pharmaceutique est administrable en perfusion intravasculaire continue.
